# EUROPEAN PATENT APPLICATION

(11) **EP 2 669 307 A1**
(43) Date of publication of application: **04.12.2013**
(21) Application number: 12170576.8
(22) Date of filing: 01.06.2012
(51) Int. Cl.: C08G 59/04, C08G 59/06, C08G 59/02, C07D 301/24

(54) **Process for manufacturing an epoxide**

(71) Applicant: SOLVAY SA, 1120 Bruxelles (BE)
(72) Inventor: Delannoy, Vincent, 7911 Oeudeghein (BE)
(74) Representative: Vande Gucht, Anne

(57) **Abstract**

Process for manufacturing an epoxide, comprising consuming a first compound containing chlorine (A), generating a second compound containing chlorine (B), converting (B) into (A), and wherein the molar ratio between consumed (A) and (A) converted from (B), expressed as elemental chlorine (Cl), is higher than or equal to 0.9.

## Description

The present invention relates to a process for manufacturing an epoxide.

The present invention relates more specifically to a process for manufacturing an epoxide wherein chlorinated compounds are used and generated.

Epoxides, like epichlorohydrin and epoxy resins, are useful intermediates in the manufacture of day life articles.

Epichlorohydrin is a reaction intermediate in the manufacture of epoxy resins, synthetic elastomeres, glycidyl ethers, polyamide resins, etc. (Ullmann's Encyclopedia of Industrial Chemistry, Fifth Edition, Vol. A9, p.539).

Epoxy resins may be used in "coating" applications or "structural" applications.

"Coating" applications may be found in the marine field (corrosion-resistant coating for boats, for example), in the field of metal containers (cans for food use, for example), in the coil coating field, and in the field of coatings for motor vehicles, to name but a few of them.

"Structural" applications may be found in the field of composites (reinforcing fibers based on glass, boron or graphite for example), in the civil engineering field, in the fields of floor coverings, of construction, of electrical laminates (printed circuits), of electrical and electronic applications (transformers and insulators, for example), of adhesives, and of tooling (prototypes and moulds, for example), to name but a few of them.

Epoxides are industrially obtained from chlorinated precursors such as dichloropropanol for epichlorohydrin and epichlorohydrin for epoxy resins. Said industrial processes generate chlorine containing co-products which have to be treated.

International application WO 2008/152043 of SOLVAY S.A. discloses such a treatment for a brine issued from a process for manufacturing epichlorohydrin.

While such treatment appears to be attractive, opportunities for improvement remain, in particular, for minimizing the chlorine containing effluents that have to be disposed of.

The present invention therefore relates to a process for manufacturing an epoxide, comprising consuming a first compound containing chlorine (A), generating a second compound containing chlorine (B), converting (B) into (A), and wherein the molar ratio between consumed (A) and (A) converted from (B), expressed as elemental chlorine (Cl), is higher than or equal to 0.9.

In the process according to the invention, compound (A) converted from (B) is preferably recycled in the said process.

One of the essential features of the present invention lies in the high conversion of (B) into (A). This has the advantage to provide a process which is self-supported in chlorine, *i.e.* with :
■ No need for an external supply of the first compound containing chlorine (A) ;
■ No chlorine containing waste to be disposed of;
■ Avoidance of handling, transportation and storage for hazardous chemicals, in particular when the compound containing chlorine (A) is hydrogen chloride ;
■ Close to zero chlorine containing effluent for the epoxide manufacturing process.

In the process according to the invention, the molar ratio between consumed (A) and (A) converted from (B), expressed as elemental chlorine (Cl), is preferably higher than or equal to 0.95 and more preferably higher than or equal to 0.99. This molar ratio is usually lower than or equal to 1.

In the process according to the invention, the epoxide is preferably selected from the group consisting of propylene oxide, epichlorohydrin, an epoxy resin or any mixture thereof.

In the process according to the invention, the epoxide is more preferably selected from the group consisting of epichlorohydrin, an epoxy resin or any mixture thereof. The epoxide is more preferably an epoxy resin.

In the process according to the invention, the expression "epoxy resin" is understood to mean a monomer or a polymer, the chemical formula of which contains at least one 2,3-epoxypropyloxy group. The epoxy resin is preferably a polymer.

Examples of monomers are for instance, monoglycidyl ethers, polyglycidyl ethers, or a mixture thereof. The polyglycidyl ether can be a diglycidyl ether, like the diglycidyl ether of bisphenol A (DGEBA) or the diglycidyl ether of isosorbide (diglycidylether of 1,4:3,6-dianhydro-D-sorbitol) for instance.

The term "polymer" is understood to mean molecules comprising several units joined to one another by covalent bonds, often in a repeating manner, these units being referred to as repeating units. The number of repeating units is greater than zero. A polymer contains at least one type of repeating unit. When the polymer contains only a single type of repeating unit, it is known as a homopolymer. When the polymer contains more than a single type of repeating unit, it is known as a copolymer. The copolymer may be of statistical, alternating or block type, as described in "Polymer Science Dictionary, M.S.M., Elsevier Applied Science, London and New York, 1989, page 86".

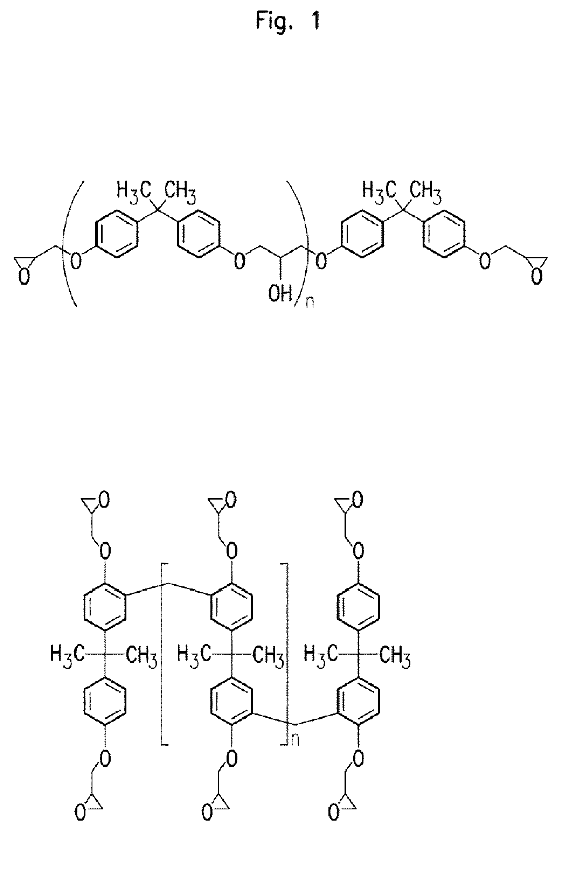

Examples of chemical formulae of epoxy resins are presented in Figure 1, when n is higher than or equal to zero and preferably higher than zero.

In the process according to the invention, the epoxy resin is preferably selected from the group consisting of Type I Grade 1 Classes A to H, Type II Grade 1 Classes A to F and Type VI Grade 1 Class A resins, as defined in the ASTM D 1763 - 00 (2005) standard entitled "Standard Specifications for Epoxy Resins", and any mixture thereof.

In the process according to the invention, the epoxy resin is more preferably a liquid epoxy resin. The expression "liquid epoxy resin" is understood to mean Type I Grade 1 Classes A and B, Type II Grade 1 Classes A, B and C, Type IV Grade 1 Classes A to D, Type V Grade 1 Classes A and B and Type VI Grade 1 Class A resins, as defined in the ASTM D 1763 - 00 (2005) standard entitled "Standard Specifications for Epoxy Resins".

In the process according to the invention, the first compound containing chlorine (A) is preferably hydrogen chloride. The hydrogen chloride may be in the form of gas or an aqueous solution of hydrogen chloride or a mixture of the two, preferably in the form of gas or a mixture of gas and an aqueous solution of hydrogen chloride.

In the process according to the invention, the second compound containing chlorine (B) is preferably a metal chloride, selected from the group consisting of an alkali metal chloride, an alkaline-earth metal chloride, or any mixture thereof. The second compound containing chlorine (B) is preferably sodium chloride.

In the process according to the invention, it is most preferred that the first compound containing chlorine (A) is hydrogen chloride and the second compound containing chlorine (B) is sodium chloride.

In the process according to the invention, the second compound containing chloride (B) can be converted into the first compound containing chloride (A) by any process. It is preferred that (B) is converted into (A) by electrolysis, in particular when (B) is a metal chloride, especially sodium chloride.

In the process according to the invention, (B) can be generated under any form, e.g. as a solid, a liquid, a gas, an aqueous composition or any combination thereof. It is preferred that (B) is generated as an aqueous composition, more preferably as a brine, in particular when (B) is a metal chloride, especially sodium chloride. The content of sodium chloride in the brine is generally higher than or equal to 10 g per kg of brine, in many cases higher than or equal to 30 g/kg, usually higher than or equal to 70 g/kg, frequently higher than or equal to 100 g/kg, often higher than or equal to 140 g/kg, in particular higher than or equal to 160 g/kg and most specifically higher than or equal to 200 g/kg. The sodium chloride content is usually lower than or equal to 350 g/kg, commonly lower than or equal to 325 g/kg, generally lower than or equal to 270 g/kg, often lower than or equal to 250 g/kg, and frequently lower than or equal to 230 g/kg. A sodium chloride content of about 200 g/kg is particularly convenient.

In the process according to the invention, when (B) is generated as a brine, in particular a brine containing a metal chloride, more specifically sodium chloride, the electrolysis can be carried out in any type of chlor-alkali electrolysis cells, in order to produce hydrogen, chlorine and a metal hydroxide, more specifically sodium hydroxide, and at least one part of the hydrogen and the chlorine are reacted to produce the hydrogen chloride (A). In a preferred aspect, hydrogen and chlorine are reacted in a molar ratio between hydrogen and chlorine lower than or equal to 1.

The chlor-alkali electrolysis cell may be selected from a chlor-alkali mercury electrolysis cell, a chlor-alkali diaphragm electrolysis cell, a chlor-alkali membrane electrolysis cell, and any combination thereof. A chlor-alkali membrane electrolysis cell is more preferred.

In the process according to the invention, when (B) is generated as a brine comprising sodium chloride, the process comprises electrolyzing said brine, preferably in a chlor-alkali membrane cell, in order to produce hydrogen, chlorine and sodium hydroxide, and reacting at least one part of said hydrogen and chlorine to produce (A).

In the process according to the invention, at least one part of the metal hydroxide, more specifically sodium hydroxide, produced by electrolysis, may be recycled in the process. It is preferred that at least one part of sodium hydroxide produced by electrolysis is recycled in the said process.

In one aspect of the process according to the invention, when the epoxide is an epoxy resin and when (B) is generated as a brine comprising sodium chloride, the process preferably comprises reacting dichloropropanol, a compound containing at least one active hydrogen atom and sodium hydroxide in order to produce the epoxy resin and sodium chloride, at least one part of the sodium hydroxide has preferably been produced by the electrolysis of the brine, and the dichloropropanol has preferably been obtained by reacting glycerol and hydrogen chloride.

In that aspect, it is preferred that at least one of the dichloropropanol and of the compound containing at least one active hydrogen atom, has been obtained from renewable raw materials.

In that aspect of the process according to the invention, the glycerol may be obtained from fossil or renewable raw materials. It is preferred to use glycerol obtained from renewable materials. A glycerol which is particularly suitable may be obtained during the conversions of fats or oils of vegetable or animal origin, such as saponification, transesterification or hydrolysis reactions. A particularly suitable glycerol may be obtained during the conversion of animal fats. Another particularly suitable glycerol may be obtained during the manufacture of biodiesel. Another particularly suitable glycerol may be obtained during the fatty acid manufacture. Another particularly suitable glycerol may be obtained during the fatty alcohols manufacture.

In that aspect of the process according to the invention, the dichloropropanol has preferably been obtained by reacting hydrogen chloride with glycerol obtained from renewable raw materials.

In that aspect, by dichloropropanol, one intends to denote any one of 1,3-dichloropropane-2-ol, 2,3-dichloropropane-1-ol, and mixture thereof. Mixtures consisting essentially of 1,3-dichloropropane-2-ol and 2,3-dichloropropane-1-ol are preferred. In the mixture of 1,3-dichloropropane-2-ol and 2,3-dichloropropane-1-ol, the 1,3-dichloro-2-propanol content with respect to the total content of the two dichloropropanol isomers is preferably higher than or equal to 100 g/kg, yet preferably higher than or equal to 300 g/kg, still preferably higher than or equal to at least 400 g/kg, more preferably higher than or equal to 750 g/kg, still more preferably higher than or equal to 800 g/kg, yet more preferably higher than or equal to 900 g/kg, and most preferably higher than or equal to 920 g/kg. This content of 1,3-dichloro-2-propanol in the mixture is generally at most 990 g/kg and usually at most 960 g/kg. Contents of 925, 930, 935, 940, 945, 950 or 955 g/kg are particularly convenient. It is also possible to use a dichloropropanol composed essentially of 1,3-dichloro-2-propanol.

In that aspect of the process according to the invention, the dichloropropanol can be obtained by reaction between glycerol and a chlorinating agent as described in Patent Applications WO 2005/054167, WO 2006/100311, WO 2006/100312, WO 2006/100313, WO 2006/100314, WO 2006/100315, WO 2006/100316, WO 2006/100317, WO 2006/106153, WO 2007/054505, WO 2006/100318, WO 2006/100319, WO 2006/100320, WO 2006/106154, WO 2006/106155, WO 2007/144335, WO 2008/107468, WO 2008/101866, WO 2008/145729, WO 2008/110588, WO 2009/000773, WO 2009/043796, WO 2009/121853, WO 2009/077528, WO 2010/066660, WO 2010/029039 and WO 2010/029153, filed in the name of SOLVAY, the contents of which are incorporated herein by reference.

In that aspect of the process according to the invention, a part of the dichloropropanol may have been derived from any other processes such as, for example, the allyl chloride hypochlorination process, the allyl alcohol chlorination process, the glycerol monochlorohydrin hydrochlorination process, the epichlorohydrin hydrochlorination process,the 2,3-dichloropropionaldehyde hydrogenation process as described in documents WO 1997/48667, US 6,350,922 and US 5,744,655, the 1,2-dichlorethylene hydroformylation process as described in document WO 2005/116004, the 1,3-dichloroacetone hydrogenation process as described in documents WO 2005/097722 and WO 2003/064357. 2,3-dichloropropionaldehyde may itself be obtained by chlorination of acrolein and/or hydroformylation of 1,2-dichloroethylene as described in documents US 2,860,146 and WO 2005/116004. 1,3-dichloroacetone may itself be obtained by chlorination of acetone and/or by bromine/chlorine exchange starting from 1,3-dibromoacetone as described in Applications WO 2005/097722 and WO 2005/115954. Acrolein may be obtained by selective oxidation of propylene. 1,2-dichloroethylene may be a by-product of the synthesis of vinyl chloride starting from ethane and/or be obtained by chlorination of acetylene. Acetylene may be obtained by conventional processes such as hydrolysis of calcium carbide and/or pyrolysis of hydrocarbons, crude oil and even coal, such as described in "Industrial Organic Chemistry, Third, Completely Revised Edition, VCH, 1997, pp. 93-98". 1,3-dibromoacetone may be obtained by bromination of acetone, as described in document WO 2005/115954. Acetone may itself be obtained by conventional processes, such as, for example, oxidation of propylene, dehydrogenation of isopropanol and/or decomposition of cumene hydroperoxide, as described in "Industrial Organic Chemistry, Third, Completely Revised Edition, VCH, 1997, pp. 276-277 and 347-355".

In that aspect of the process according to the invention, the compound containing at least one active hydrogen atom can be of any type. This compound may have been from renewable raw materials, fossil raw materials and any combination thereof. This compound is preferably obtained from renewable raw materials.

The compound containing at least one active hydrogen atom is preferably selected from the group consisting of a polyol, a polycarboxylic acid, a polyamine, an amino alcohol, a polyimide, a polyamide, a polyaminoamide, a polyimine, an acid mono- or polyphenol and mixtures of at least two of these compounds.

In the process according to the invention, the compound containing at least one active hydrogen atom is more often a polyol selected from the group constituted of bisphenol A (4,4'-dihydroxy-2,2-diphenylpropane, 4,4'-isopropylidenediphenol), tetrabromobisphenol A (4,4'-isopropylidenebis(2,6-dibromophenol)), bisphenol AF (4,4'-[2,2,2-trifluoro-1-(trifluoromethyl)ethylidene]bisphenol), hexafluorobisphenol A (4,4'-dihydroxy-2,2-diphenyl-1,1,1,3,3,3-hexafluoropropane), 1,1,2,2-tetra(p-hydroxyphenyl)ethane, tetramethylbisphenol (4,4'-dihydroxy-3,3',5,5'-tetramethyl bisphenol), 1,5-dihydroxynaphthalene, 1,1',7,7'-tetrahydroxydinaphthylmethane, 4,4'-dihydroxy-α-methylstilbene, a condensation product of bisphenol A with formaldehyde (bisphenol A novolac), a condensation product of phenol with formaldehyde, preferably bisphenol F (mixture of o,o', o,p' and p,p' isomers of dihydroxydiphenylmethane), a condensation product of cresol with formaldehyde (mixture of o,o', o,p' and p,p' isomers of methylhydroxydiphenylmethane), an alkylation product of phenol and of dicyclopentadiene (2,5-bis[hydroxyphenyl]octahydro-4,7-methano-5H-indene), a condensation product of phenol and of glyoxal (tetrakis(4-hydroxyphenyl)ethane), a condensation product of phenol and of a hydroxybenzaldehyde (e.g. tris(4-hydroxyphenyl)methane), 1,1,3-tris(p-hydroxyphenyl)propane, and mixtures of at least two of them, or p-aminophenol.

In the process according to the invention, the compound containing at least one active hydrogen atom is more frequently a polyol, preferably containing more than three carbon atoms, selected from the group consisting of a polyphenol, a sugar, a polyol derived from a sugar, an acid polyphenol, any derivative thereof, and any mixture thereof. The polyol may be as described in international application EP2011/066689 filed in the name of SOLVAY S.A., the content of which is incorporated herein by reference, more specifically the passages from page 12, line 5, to page 16, line 13.

In the process according to the invention, when the polyol is a product derived from a sugar, the product derived from a sugar is preferably selected from the group consisting of an anhydrosugar, a reduction product from sugar, a reduction product of hydroxymethylfurfural, a difuran derivative of furfural and any mixture thereof.

In the process according to the invention, when the product derived from a sugar is an anhydrosugar, it is preferably selected from the group consisting of isosorbide, isomannide, isoidide and any mixture thereof. The anhydrosugar is more preferably isosorbide.

In a preferred variant of that aspect of the process according to the invention, epichlorohydrin is generated in situ during the reaction, generating the epoxy resin and the brine, at least one part of said epichlorohydrin is recovered and at least one fraction of the recovered epichlorohydrin is recycled to the process for manufacturing the epoxy resin.

In that variant, the process comprises at least one liquid-liquid phase separation step to remove at least one part of the epichlorohydrin formed in situ.

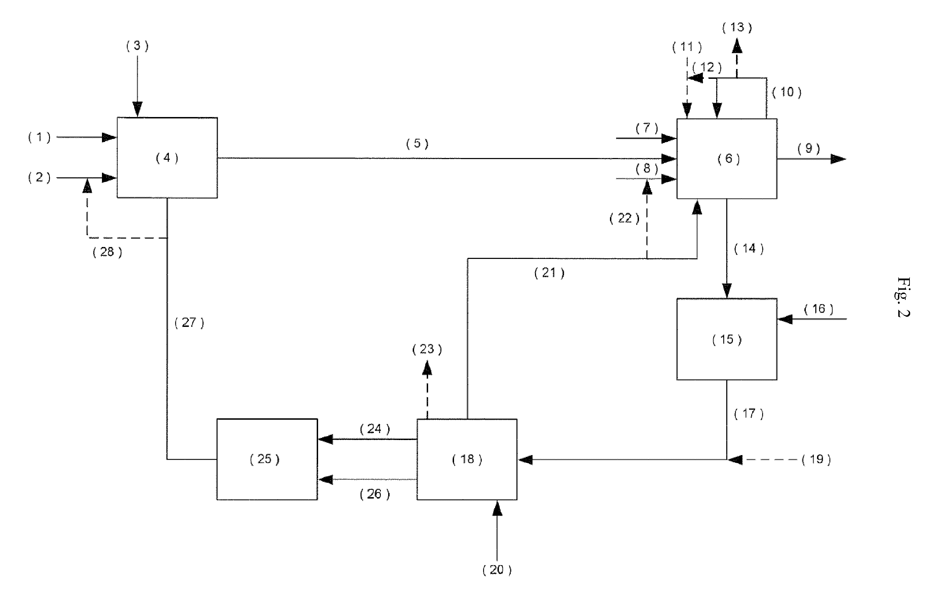

Figure 2 represents a non-limiting embodiment of the process according to the present invention.

A first stream of glycerol, preferably obtained from renewable raw materials, is fed to a first vessel (4) via a first line (1). A second stream of hydrogen chloride, gaseous, as an aqueous solution or a mixture thereof, is fed to first vessel (4) via a second line (2). A third stream of a catalyst, preferably a carboxylic acid is fed to first vessel (4) via a third line (3). First vessel (4) may comprise any combination of reactors and separation equipment. A fourth stream of dichloropropanol is withdrawn from first vessel (4) via a fourth line (5) and is fed to a second vessel (6). Also introduced in second vessel (6) are a fifth stream of a compound containing at least one active hydrogen atom via a fifth line (7) and a sixth stream of sodium hydroxide via a sixth line (8). A seventh stream of epoxy resin is withdrawn from second vessel (6) via a seventh line (9) which may be sent to storage, to further processing such as purification, or to other equipment for further reaction. An eight stream of epichlorohydrin formed in situ in second vessel (6) is withdrawn from second vessel (6) and recycled to second vessel (6) via an eighth line (10). A ninth stream of epichlorohydrin not formed in second vessel (6) may also be fed to second vessel (6) via a ninth line (11). Part of the eighth stream of epichlorohydrin may be added as a tenth stream to the ninth stream of epichlorohydrin via a tenth line (12). Part of the eight stream of the recycled epichlorohydrin may be withdrawn as an eleventh stream via a eleventh line (13) and may be sent to storage, to further processing such as purification, or to other equipment for further reaction. A twelfth stream of brine is withdrawn from second vessel (6) via a twelfth line (14) and fed to a third vessel (15). Third vessel (15) is also fed with a thirtheenth stream containing an oxidant, preferably "active chlorine" via a thirtheenth line (16). The expression "active chlorine" is understood to mean molecular chlorine and its reaction products with water, chloride ions or with a basic agent, such as hypochlorous acid, trichloride ion and sodium hypochlorite for example. A fourtheenth stream of brine is withdrawn from third vessel (16) via a fourtheenth line (17) and fed to a fourth vessel (18). A fifteenth stream of brine may be supplied via a fifteenth line (19). Also supplied to fourth vessel (18) is a sixteenth stream of electricity via a sixteenth line (20). A seventeenth stream of sodium hydroxide is withdrawn from fourth vessel (18) via a seventeenth line (21) and fed to second vessel (6). Part of the seventeenth stream of sodium hydroxide may be added as an eighteenth stream to the sixth stream of sodium hydroxide via a eighteenth line (22). A part of the seventeenth stream of sodium hydroxide may be withdrawn as a nineteenth stream via a nineteenth line (23). A twentieth stream of hydrogen is withdrawn from fourth vessel (18) via a twentieth line (24) and fed to a fifth vessel (25). Also withdrawn from fourth vessel (18) and fed to fifth vessel (25) is a twenty-first stream of chlorine via an twenty-first line (26). A twenty-second stream of hydrogen chloride is withdrawn from fifth vessel (25) and fed to first vessel (4) via a twenty-second line (27). Part of the twenty-second stream of hydrogen chloride may be added to the second stream of hydrogen chloride as a twenty-third stream via a twenty-third line (28).

Vessels (4), (6), (15), (18) and (25) contain reaction vessel of any well-known suitable type, including for example, one or more discontinuous or continuous reactors, and separation vessel of any well-known suitable type, including for example, one or more distillation columns, evaporators, strippers, settlers, adsorbers, absorbers, ion exchange equipments or combination thereof.

More specifically, vessel (18) contains any type of well-known electrolysis suitable cells, including for example, mercury, diaphragm and membrane chlor-alkali electrolysis cells.

## Claims

1. Process for manufacturing an epoxide, comprising consuming a first compound containing chlorine (A), generating a second compound containing chlorine (B), converting (B) into (A), and wherein the molar ratio between consumed (A) and (A) converted from (B), expressed as elemental chlorine (Cl), is higher than or equal to 0.9.

2. Process according to claim 1, wherein compound (A) converted from (B) is recycled to the process.

3. Process according to claim 1 or 2, wherein the molar ratio between consumed (A) and (A) converted from (B), expressed as elemental chlorine (Cl), is higher than or equal to 0.99 and lower than or equal to 1.

4. Process according to any one of claims 1 to 3, wherein the epoxide is epichlorohydrin or an epoxy resin, preferably an epoxy resin.

5. Process according to any one of claims 1 to 4, wherein the first compound containing chlorine (A) is hydrogen chloride and the second compound containing chlorine (B) is sodium chloride.

6. Process according to any one of claims 1 to 5, wherein (B) is converted into (A) by electrolysis.

7. Process according to any one of claim 5, wherein (B) is generated as a brine comprising sodium chloride, comprising electrolyzing said brine, preferably in a chlor-alkali membrane cell, in order to produce hydrogen, chlorine and sodium hydroxide, and reacting at least one part of said hydrogen and chlorine to produce the hydrogen chloride (A).

8. Process according to claim 7, wherein at least one part of the hydrogen chloride has been obtained by reacting hydrogen and chlorine, in a molar ratio between hydrogen and chlorine lower than or equal to 1.

9. Process according to claim 7 or 8, comprising recycling at least one part of sodium hydroxide in the said process.

10. Process according to claim 4, 7 or 8, wherein the epoxide is an epoxy resin, comprising reacting dichloropropanol, a compound containing at least one active hydrogen atom and sodium hydroxide in order to produce the epoxy resin and sodium chloride, wherein at least one part of the sodium hydroxide has been produced by the electrolysis of the brine, and wherein the dichloropropanol has been obtained by reacting glycerol and hydrogen chloride.

11. Process according to claim 10, wherein epichlorohydrin is generated in situ during the reaction, generating the epoxy resin and the brine, preferably comprising recovering at least one part of the epichlorohydrin and recycling at least one fraction of the recovered epichlorohydrin to the said process.

12. Process according to claim 11, comprising at least oneliquid-liquid phase separation step to remove at least one part of the epichlorohydrin formed in situ.

13. Process according to any one of claims 4 to 11 wherein the epoxy resin is selected from the group consisting of Type I Grade 1 Classes A to H, Type II Grade 1 Classes A to F and Type VI Grade 1 Class A resins, as defined in the ASTM D 1763 - 00 (2005) standard entitled "Standard Specifications for Epoxy Resins", and any mixture thereof, and wherein the epoxy resin is a liquid epoxy resin selected from the group consisting of Type I Grade 1 Classes A and B, Type II Grade 1 Classes A, B and C and Type VI Grade 1 Class A resins, as defined in the ASTM D 1763 - 00 (2005) standard entitled "Standard Specifications for Epoxy Resins" and any mixture thereof.

14. Process according to any one of claims 10 to 13 wherein the compound containing at least one active hydrogen atom is a polyol selected from the group constituted of bisphenol A (4,4'-dihydroxy-2,2-diphenylpropane, 4,4'-isopropylidenediphenol), tetrabromobisphenol A (4,4'-isopropylidenebis(2,6-dibromophenol)), bisphenol AF (4,4'-[2,2,2-trifluoro-1-(trifluoromethyl)ethylidene]bisphenol), hexafluorobisphenol A (4,4'-dihydroxy-2,2-diphenyl-1,1,1,3,3,3-hexafluoropropane), 1,1,2,2-tetra(p-hydroxyphenyl)ethane, tetramethylbisphenol (4,4'-dihydroxy-3,3',5,5'-tetramethyl bisphenol), 1,5-dihydroxynaphthalene, 1,1',7,7'-tetrahydroxydinaphthylmethane, 4,4'-dihydroxy-a-methylstilbene, a condensation product of bisphenol A with formaldehyde (bisphenol A novolac), a condensation product of phenol with formaldehyde, preferably bisphenol F (mixture of o,o', o,p' and p,p' isomers of dihydroxydiphenylmethane), a condensation product of cresol with formaldehyde (mixture of o,o', o,p' and p,p' isomers of methylhydroxydiphenylmethane), an alkylation product of phenol and of dicyclopentadiene (2,5-bis[hydroxyphenyl]octahydro-4,7-methano-5H-indene), a condensation product of phenol and of glyoxal (tetrakis(4-hydroxyphenyl)ethane), a condensation product of phenol and of a hydroxybenzaldehyde (e.g. tris(4-hydroxyphenyl)methane), 1,1,3-tris(p-hydroxyphenyl)propane, and mixtures of at least two of them, or p-aminophenol, or wherein the compound containing at least one active hydrogen atom is a polyol selected from the group consisting of a polyphenol, a sugar, a polyol derived from a sugar, preferably isosorbide, isomannide, isoidide and any mixture thereof, an acid polyphenol, any derivative thereof, and any mixture thereof.

15. Process according to any one of claims 10 to 14, wherein at least one of the dichloropropanol and of the compound containing at least one active hydrogen atom, has been obtained from renewable raw materials.
